# EUROPEAN PATENT SPECIFICATION

(11) **EP 2 417 441 B1**
(45) Date of publication and mention of the grant of the patent: **30.05.2018**
(21) Application number: 09776515.0
(22) Date of filing: 06.04.2009
(51) Int. Cl.: G01N 27/12, G01N 27/414, G01N 33/00

(54) **GAS SENSOR**
GASSENSOR
DÉTECTEUR DE GAZ

(43) Date of publication of application: 15.02.2012
(73) Proprietor: Sensic Ab, 164 40 Kista (SE)
(72) Inventor: ANDERSSON, Mike, 587 34 Linköping (SE)
(74) Representative: Awapatent AB
(86) International application number: PCT/EP2009/002528
(87) International publication number: WO 2010/115434

(56) References cited:
- DE-A1-102007 040 726
- US-A1- 2005 097 941
- SHIMANOE K ET AL: "Development of FET-type CO2 sensor operative at room temperature" SENSORS AND ACTUATORS B, ELSEVIER SEQUOIA S.A., LAUSANNE, CH, vol. 102, no. 1, 1 September 2004 (2004-09-01), pages 14-19, XP004534532 ISSN: 0925-4005
- MIKE ANDERSSON ET AL: "Detecting non-hydrogen containing species with field effect devices" SENSORS, 2008 IEEE, IEEE, PISCATAWAY, NJ, USA, 26 October 2008 (2008-10-26), pages 1320-1323, XP031375326 ISBN: 978-1-4244-2580-8
- ERIKSSON MATS ET AL: "The influence of the insulator surface properties on the hydrogen response of field-effect gas sensors", JOURNAL OF APPLIED PHYSICS, AMERICAN INSTITUTE OF PHYSICS, US, vol. 98, no. 3, 3 August 2005 (2005-08-03) , pages 34903-034903, XP012078318, ISSN: 0021-8979, DOI: 10.1063/1.1994941

## Description

### Field of the invention

The present invention relates to the field of gas sensors. In particular, the invention relates to the field of field effect gas sensors and detection of at least one non-hydrogen containing substance in a gas comprising hydrogen or hydrogen-containing substances.

### Background of the invention

Combustion processes in e.g. internal combustion car engines, power plants, local heating plants, gas turbines, and domestic heating facilities generally lead to emissions of substances such as nitrogen oxides, hydrocarbons and carbon monoxide (CO), especially if the processes are neither optimized nor controlled. Deficiency or too much of excess air in the combustion processes lead to either incomplete combustion of the fuel or slow combustion kinetics, with the result that incompletely oxidized hydrocarbon species and CO are left in the exhaust or flue gases. Generally, combustion processes also lead to generation of nitrogen oxides and release of fuel-bound nitrogen and sulphur oxides, the emissions of which normally are reduced by post-combustion processes, e.g. catalytic conversion and wet scrubbing.

Optimizing and controlling a combustion process, as well as any post-combustion measures, in order to decrease the emissions requires monitoring and determination of certain gaseous substances, such as CO, NO, NO₂, in the exhaust or flue gases. The presently existing options regarding such monitoring and determination is however very limited due to the harsh conditions, e.g. high temperature, vibrations, corrosive environments, encountered in the processes of interest. Most solid-state gas sensors are either not able to operate under harsh conditions or suffer long-term stability problems. As an example, in the special application of Exhaust Gas Recirculation (EGR), there is at present no existing satisfactory intake oxygen sensor for control of the exhaust recirculation. The Universal Exhaust Gas Oxygen (UEGO) sensor currently in use for exhaust or flue gas oxygen concentration assessment does not withstand the conditions encountered. Other kinds of sensor technologies require the gas to be sampled, cooled and/or filtered before being subjected to the sensors, such as the sensor technology based on electrochemical cells. Sensors based on solid electrolytes are quite expensive and, for most of the interesting target substances, do not provide the accuracy needed for e.g. control of exhaust after-treatment systems. The various kinds of optical sensors that have been developed are also quite expensive and suffer from undesired spatial fluctuations when directing the laser beam of the sensor to desired locations (also referred to as "beam wobble" or "pointing instability"), as well as long-term stability issues.

Gas sensors utilizing field effect devices are promising for measuring important exhaust/flue gas constituents or other gas compositions from high temperature or harsh environment processes, e.g. the sensor device disclosed in US 7053425. The basic design of a field effect gas sensitive device is also given in e.g. Savage, S. M. et al. Mater. Sci. Forum, 353-356 (2001), pp. 747-752.

A few examples of prior art sensors are shown in:
Shimanoe, K. et al. Sensors and Actuators, B102 (2004), 14-19, which relates to a SiC based MISFET device employing a gate material combination comprising an ion conducting (Li₂ₓBa₁₋ₓCO₃), an ion exchange (NASICON) and a conducting (ITO) material on top of a passivation layer (Ta₂O₅) for CO₂ detection.

Andersson, M. et. al. Sensors (2008), 1320-1323, which relates to a MOSFET/MISFET gas sensor comprising an insulator layer composed of LPCVD deposited and densified nitride(Si₃N₄).

US 2005/097941 A1, which relates to a general MOSFET/MISFET device for gas detection comprising electrical passivation layers having a layer made from e.g. thermally grown SiO₂.

DE 10 2007 040726 A1 relates to MOSFET/MISFET devices having increased selectivity due to the presence of catalytic filters. Any reduction of cross-sensitivity to e.g. hydrogen or hydrogen containing substances is provided by the catalytic filter.

Generally, the sensing mechanism in a field effect gas sensor based on a transistor is achieved as follows: A voltage is applied between the source and drain contacts and causes a current to flow through the channel region. A material capable of interacting with the substance or substances of interest in such a way that the electric field from the gate to the semiconductor is changed upon the interaction is used as the gate contact of the device, and is placed on top of the insulating layer over the channel region. The electric field from this gate contact to the semiconductor in turn modulates the current in the channel. As an example, if the field effect gas sensor is used for detecting H₂-gas, the gate contact is chosen to facilitate dissociative adsorption of the hydrogen molecule on its surface, producing hydrogen atoms that rapidly diffuse through the metal gate contact, adsorbing at the metal/insulator interface in the form of polarized hydroxyl (-OH) groups on the oxide surface. This polar layer at the interface changes the electric field from the contact and thus the current through the channel in such way that the change in current reflects the hydrogen coverage at the interface, which is directly related to the ambient hydrogen concentration. Such field effect devices can thus provide a good platform for the detection of various gaseous substances in applications involving high temperature processes and corrosive atmospheres.

However, traditional field effect sensor devices developed from this platform are all inherently sensitive to hydrogen and certain hydrogen-containing species, due to the chemisorption of hydrogen at the metal contact/insulator interface and the resulting polarized layer of hydroxyl groups. See for example Eriksson, M. et. al., Journal of Applied Physics, 98 (2005), 034903 1-6, wherein the influence of the choice of insulator of a MIS field-effect gas sensor on the hydrogen response detection limit is investigated. Consequently, detection of non-hydrogen containing species, e.g. O₂, CO, NO, NO₂, SO₂, with these traditional kind of field effect devices severely suffer from cross-sensitivity problems to hydrogen and hydrogen-containing species. The possibility to detect non-hydrogen containing species with the field effect sensors is thus very limited, since hydrogen and/or hydrogen-containing species are present in the above-mentioned applications, such as in combustion processes.

Thus, there is a need in the art for a gas sensor device for monitoring and quantifying non-hydrogen containing substances in a gas comprising hydrogen or hydrogen-containing substances in a number of specific applications where high temperatures, vibrations and harsh environments are encountered.

### Summary of the invention

An object of the present invention is to provide improved means for detecting non-hydrogen containing substances in a gas.

A further object of the present invention is to provide means for improving the signal-to-noise ratio when detecting at least one non-hydrogen containing substance in a gas comprising hydrogen or hydrogen-containing substances.

Yet another object of the present invention is to provide a gas sensor that facilitates control of Exhaust Gas Recirculation (EGR) in diesel engines, thus lowering emissions of e.g. nitrogen oxides.

The above-mentioned objects as well as other objects of the invention are met by the different aspects of the disclosed invention.

The present invention is defined by the appending claims.

In a first aspect of the present invention, there is provided a field effect gas sensor for detection of at least one non-hydrogen containing substance in a gas comprising hydrogen or hydrogen-containing substances, the field effect gas sensor comprising:
- a semiconductor layer having a surface;
- at least one ohmic contact contacting the semiconductor;
- a first electron insulating layer covering at least a part of the surface of the semiconductor layer;
- a second insulating layer comprising MgO, the second insulating layer contacting at least one of the first electron insulating layer and the semiconductor layer;
- at least one electrical contact contacting the second insulating layer and comprising a conducting, semi-conducting and/or ion conducting layer;
wherein the field effect gas sensor is configured so interaction of the at least one non-hydrogen containing substance with at least part of the at least one electrical contact affects the work function of the conducting, semi-conducting and/or ion conducting layer and/or the electric field in the semiconductor layer in such way that the current-voltage (I-V) or the capacitance-voltage (C-V) characteristics of the field effect gas sensor are changed, wherein the change in I-V or C-V characteristics provide information about the presence of the at least one non-hydrogen containing substance in the gas.

A field effect gas sensor refers to any type of field effect electronic device in which at least one electric characteristic of the device, such as a work function or an electric field, changes as a response to one or several specific molecules in the ambient environment.

A semiconductor layer refers to a layer of at least one solid material having an electrical conductivity that can vary over a wide range, either permanently or dynamically. The semiconductor layer may also be doped, and the doping may be different in different parts of the semiconductor layer. Further, the semiconductor layer may include one or more epitaxial layers, i.e. layers deposited/grown on top of or on the surface of the semiconductor layer. The epitaxial layer(s) may also be doped, and the doping may be different in different parts of the epitaxial layer(s).

An ohmic contact refers to a metal-semiconductor contact with very low resistance independent of applied voltage, i.e. a contact having no or a very small potential barrier at the metal-semiconductor interface. The at least one ohmic contact may comprise several materials and may be arranged as a stack of different layers or materials to give a better stability towards the ambient gas mixture at elevated temperatures and to facilitate easy bonding to e.g. wires or circuit boards. The at least one ohmic contact may be processed by depositing the contact material or contact materials of the at least one ohmic contact at regions where a pre-deposited electron insulating layer has been etched by e.g. standard photo-lithographic patterning and wet etching techniques.

An electron insulating layer refers to a material that does not conduct electrons, i.e. an insulator. Such insulating layers are known to a person skilled in the art of semiconductor technology.

The insulating layer comprises a material substantially chemically inert to hydrogen or hydrogen-containing substances and is an insulating layer comprising a material or combination of materials that is either never hydrogen terminated at any instant, no matter the ambient gas composition, or has a very weak hydrogen coverage dependence on the ambient concentration of hydrogen or hydrogen-containing substances.

An electrical contact refers to a contact that is capable of conducting or semiconducting electrons or ions.

The work function of the conducting, semi-conducting and/or ion conducting layer refers to the minimum energy required to remove an electron from the conducting, semi-conducting and/or ion conducting layer to a point immediately outside the layer, i.e. the energy required to move an electron in the conducting, semi-conducting and/or ion conducting layer from the Fermi level to vacuum.

The gas sensor according to the present invention has a configuration so that interaction of the gas with at least part of the at least one electrical contact affects the work function of the conducting, semi-conducting and/or ion conducting layer and/or the electric field in the semiconductor layer in such way that the current-voltage (I-V) or the capacitance-voltage (C-V) characteristics of the field effect gas sensor are changed. The changed I-V or C-V characteristics provide information about the presence of the at least one non-hydrogen containing substance in the gas and refers to the sensing mechanism of the sensor. The changed I-V or C-V characteristics may hence be used as a sensor signal providing information of the at least one non-hydrogen containing substance in the gas. The information may for example be an increased or decreased amount of the at least one non-hydrogen containing substance in the ambient or a concentration of at least one non-hydrogen containing substance in the ambient. Consequently, interaction of the at least one non-hydrogen containing substance in a gas comprising hydrogen or hydrogen-containing substances with at least part of the at least one electrical contact of the sensor changes the I-V or C-V characteristics of the sensor, providing an electrical signal that gives information about the presence and possibly also quantity and/or concentration of these substances in the ambient. The I-V and C-V characteristics of a field effect device is a property well-known to a person skilled in the art.

The first aspect of the invention is based on the insight that a second insulating layer comprising MgO significantly enhances the specificity and sensitivity for a field effect gas sensor towards at least one non-hydrogen containing substance in a gas comprising hydrogen or hydrogen-containing substances.

Thus, reduced hydrogen adsorption at the electrical contact/second insulator interface reduces the influence of hydrogen and hydrogen-containing substances on the sensor signal, leading to a better signal-to-noise ratio. Further, the specificity of the sensor to the at least one non-hydrogen containing substance is also increased due to the at least one electrical contact comprising a conducting, semi-conducting and/or ion conducting layer that may change its work function upon interaction with the at least one non-hydrogen containing substance. Moreover, the design of the field effect gas sensor according to the first aspect of the invention also provides better signal stability as compared to e.g. resistive type of devices composed of the same materials. Consequently, these advantages facilitate better sensitivity and selectivity to the non-hydrogen containing contents in the gases of interest, giving better possibilities for reaching the requirements from applications such as on-board diagnostics (OBD) in cars, control of exhaust gas recirculation (EGR) in diesel cars, combustion control in local and domestic heating systems etc.

In an embodiment of the first aspect, the I-V or the C-V characteristics are changed such that a voltage applied over the at least one ohmic contact to keep a constant current through or a constant capacitance over the semiconductor layer increases or decreases, wherein the increase or decrease provides information about the presence of the at least one non-hydrogen containing substance in the gas.

A voltage applied over the at least one ohmic contact to keep a constant current through or a constant capacitance over the semiconductor layer is a convenient electrical signal that may provide information about the presence of the at least one non-hydrogen containing substance in the gas. Upon interaction with the at least one non-hydrogen containing substance, the applied voltage may increase or decrease as the concentration of the at least one non-hydrogen containing substance in the gas increases. If the applied voltage increases or decreases may depend on the nature of the at least one non-hydrogen containing substance. Consequently, the applied voltage may also increase or decrease as the concentration of the at least one non-hydrogen containing substance in the gas decreases.

In an embodiment according to the first aspect of the invention, the field effect gas sensor is selected from the group consisting of MIS/MOS (Metal Insulator Semiconductor/ Metal Oxide Semiconductor) capacitors, Schottky diodes and field effect transistors.

These types of electrical field effect components have well studied current-voltage or capacitance-voltage characteristics and may thus be suitable components as the gas sensor of the present disclosure.

In another embodiment of the first aspect, the field effect transistor is selected from the group consisting of Metal Oxide Semiconductor Field Effect Transistors (MOSFET), Metal Insulator Semiconductor Field Effect Transistors (MISFET), Metal Semiconductor Field Effect Transistors (MESFET), Heterostructure Field Effect Transistors (HFET), and Metal Insulator Semiconductor Heterostructure Field Effect Transistors (MISHFET).

Further, if the gas sensor is a field effect transistor, the gas sensitive electrical contact of the gas sensor may be the gate contact of the field effect transistor, and the ohmic contacts may be the source and drain contact, respectively.

In another embodiment of the first aspect, the at least one non-hydrogen containing substance is selected from CO, CO₂, NO, NO₂, N₂O, SO₂, SO₃ and O₂, or any combination thereof.

In industrial applications, these non-hydrogen compounds are often found together with hydrogen or hydrogen-containing substances in gases. By using different materials in the electrical contact of the field effect gas sensor, the selectivity and sensitivity to different target species can be tuned. Consequently, the detection and/or quantification of one or more of the above listed non-hydrogen-containing substances may also be obtained from the combined signal from two or more field effect gas sensors of the present disclosure with different gas sensitive electrical contacts.

In a further embodiment of the first aspect, the semiconductor layer comprises a wide band gap semiconductor.

A band gap refers to the energy difference between the top of the valence band and the bottom of the conduction band of the semiconductor, i.e. the amount of energy required to remove an outer shell electron from an atomic nucleus in the semiconductor. A wide band gap semiconductor refers to a semiconductor having a band gap that is larger than one electron volt.

Field effect devices based on wide band gap semiconductors are due to their electrical characteristics and chemical inertness suitable for operation in high temperature applications and applications involving corrosive conditions. Field effect devices based on wide band gap semiconductors can also be manufactured on a large scale by traditional semiconductor processing technology. Furthermore, wide band gap semiconductors are stable also at elevated temperatures, and therefore the reproducibility and cost-effectiveness as well as the simple read-out may make gas sensors according to the present disclosure having a semiconductor layer comprising a wide band gap semiconductor suitable for applications where presently there are no real alternatives, e.g. in the control of exhaust gas recirculation (EGR) in diesel cars.

In an embodiment of the first aspect of the invention, the wide band gap semiconductor is selected from the group consisting of SiC, group III nitrides and alloys of group III nitrides, such as, GaN, Al_{X}Ga_{1-X}N, AlN, InN, In_{X}Ga₁-_{X}N and ZnO, or any combination thereof.

These wide band gap semiconductor materials are suitable for use at high temperatures and in corrosive environments.

According to an embodiment of the first aspect of the invention, the first electron insulating layer comprises a material selected from the group consisting of SiO₂, Al₂O₃, Ta₂O₅ and V₂O₅, or any combination thereof.

The first electron insulating layer may comprise several materials arranged e.g. as a stack structure. The first electron insulating layer may cover different parts of the surface of the semiconductor. Furthermore, the first electron insulating layer may comprise one or more passivation layers. A passivation layer refers to a layer in which chemically and electrically active bonds are saturated and thus deactivated, which means that the passivation layer is rendered chemically "passive". Examples of suitable materials as passivation layers are Si₃N₄ and AIN.

MgO has in theoretical calculations and in experiments shown to be almost totally inert regarding hydrogen, as shown in the Experimental example of the present disclosure.

MgO may be deposited e.g. by RF or reactive sputtering in an oxygen ambient, or by evaporation. The thickness of the MgO may be between 100 Å and 1000 Å, such as approximately 500 Å.

In a further embodiment of the first aspect of the invention, the conducting, semi-conducting and/or ion conducting layer of the electrical contact conducts ions of the at least one non-hydrogen containing substance. As an example, if the gas sensor is adapted for detecting oxygen, the electrical contact may comprise a material that conducts oxygen ions, and be insulating regarding conduction of electrons.

In another embodiment of the first aspect, the conducting, semi-conducting and/or ion conducting layer comprises a material selected from the group consisting of Pt, Ir, Ru, Rh, RuO₂, IrO₂, La₁₋ₓBaₓCoO₃, CeO₂, SnO₂, WO₃, TiO₂ and conducting ceramics, or any combination thereof. As an example, if the gas sensor is adopted for detecting oxygen, the electrical contact may comprise RuO₂ or IrO₂, in which the work function changes with the oxygen content, i.e. the work function changes with oxidation state. The conducting, semi-conducting and/or ion conducting layer may thus comprise one or several of the above mentioned materials, in order to tune the sensitivity against different non-hydrogen containing substances in the gas. The electrical contacts may thus be made as a layered structure comprising more than one kind of conducting, semi-conducting and/or ion conducting material, each layer from a different material.

The first electron insulating layer, the second insulating layer and the conducting, semi-conducting and/or ion conducting layer may be deposited via any known method, e.g. sputtering, thermal evaporation, e-beam evaporation, plasma enhanced chemical vapour deposition (PECVD), pulsed laser deposition (PLD), low pressure chemical vapour deposition (LPCVD), molecular beam epitaxy (MBE), spin-coating, and any combinations thereof, to thicknesses ranging from about 25 to approximately 5000 Å. The interaction of the at least one non-hydrogen containing substance with at least part of the at least one electrical contact is selected from the group consisting of adsorption of the at least one non-hydrogen containing substance on the electrical contact, absorption of the at least one non-hydrogen containing substance into the electrical contact and a chemical reaction involving the at least one non-hydrogen containing substance on the electrical contact, or any combination thereof.

Adsorption of the at least one non-hydrogen containing substance on the electrical contact refers to the accumulation of the at least one non-hydrogen containing substance on the surface of the electrical contact. Absorption of the at least one non-hydrogen containing substance into the electrical contact refers to the penetration of the at least one non-hydrogen containing substance into at least part of the electrical contact. A chemical reaction involving the at least one non-hydrogen containing substance on the electrical contact refers to a chemical reaction either on the surface of the electrical contact or in the electrical contact. The chemical reaction may be an oxidation or reduction or a reaction in which non-hydrogen containing molecules of the at least one non-hydrogen containing substance are split.

Adsorption of the at least one non-hydrogen containing substance on the electrical contact may lead to absorption of the substance into the electrical contact, thereby facilitating diffusion through the electrical contact and subsequent adsorption of the substance at the interface between the electrical contact and the first and/or second insulating layer, which may affect the work function of said conducting, semi-conducting and/or ion conducting layer and/or the electric field in said semiconductor layer.

Further, a chemical reaction involving the at least one non-hydrogen containing substance on the electrical contact may lead to reaction products that may adsorb on the first and/or second insulating layer or absorb into the electrical contact, which may affect the work function of said conducting, semi-conducting and/or ion conducting layer and/or the electric field in said semiconductor layer.

As an example, if the sensor is adapted for detecting oxygen, the chemical reaction may be the splitting of oxygen into oxygen ions, i.e. the conversion of O₂ to oxygen ions. As another example, if the sensor is adapted for detecting NO, the chemical reaction may be the oxidation of NO to NO₂ on the surface of the electrical contact.

In an embodiment of the first aspect, the at least one electrical contact comprises at least one catalytic material. In another embodiment of the first aspect of the invention, the field effect gas sensor further comprises at least one catalytic material contacting at least one of the first electron insulating layer, the second insulating layer or the at least one electrical contact.

A catalytic material refers to a material that catalyses a chemical reaction. If the at least one electrical contact comprises at least one catalytic material, the catalytic material may catalyze a chemical reaction involving the at least one non-hydrogen containing substance, such as the chemical reactions mentioned above. Further, the interaction of the at least one non-hydrogen containing substance with at least part of the at least one electrical contact may be an interaction with the catalytic material, such as adsorption of the at least one non-hydrogen containing substance on the catalytic material, absorption of the at least one non-hydrogen containing substance into the catalytic material or a chemical reaction involving the at least one non-hydrogen containing substance on the catalytic material.

In an embodiment of the first aspect of the invention, the at least one catalytic material is porous.

A porous material may facilitate the passage of the at least one non-hydrogen containing substance into the catalytic material, thus allowing a larger amount of the at least one non-hydrogen containing substance to adsorb on the surface of the catalytic material or absorb into the catalytic material. Further, a porous catalytic material may facilitate a chemical reaction involving the at least one non-hydrogen containing substance to take place at the boundary between the catalytic material and the first or second insulating layer. In this way, the selectivity of the gas sensor may be tuned by the use of a porous material. As an example, an electrical contact of porous iridium contact may be very sensitive against ammonia.

Below follows non-claimed aspects of the invention.

The at least one catalytic material may be selected from Pt, Rh, Ir and Ru, or any alloy or combination thereof. The selectivity of the gas sensor may be tuned by selecting different catalytic materials. Further, the at least one catalytic material may comprise both a porous material and a non-porous material.

The at least one ohmic contact may comprise at least one material selected from the group consisting of Ni, Ti, Au, Pt, TaSi₂, Ti₃SiC₂, Pd and any combinations or layered structures thereof.

These materials are suitable as at least part of ohmic contacts. As mentioned above, the at least one ohmic contact may be arranged as a stack of different layers or materials to give a better stability towards the ambient gas mixture at elevated temperatures and to facilitate easy bonding to e.g. wires or circuit boards.The field effect gas sensor may further comprise means for encapsulation. The encapsulation may be in such way that parts of the gas sensor that do not have to be in contact with the at least one non-hydrogen containing substance are at least partly covered. The encapsulation may protect these parts from physical impact of soot and ash particles, water droplets and oil, as well as corrosive substances. The encapsulation layer may be e.g. a thick, dense layer of SiO₂, Si₃N₄, a ceramic, or a combination thereof, and designed such that at least a portion of the at least one electrical contact remains exposed to the ambient gases. As an example, the field effect gas sensor may be encapsulated so that only the at least one electrical contact and possibly part of the at least one ohmic contact are in contact with the ambient air.

The field effect gas sensor may further comprise means for heating and controlling the temperature of the field effect gas sensor.

Means for heating and controlling the temperature of the field effect gas sensor provides the possibility of controlling and adjusting the operation temperature of the device, thereby offering the possibilities of stable operation in ambients with varying temperature and/or better discrimination, accuracy and/ or sensitivity towards a certain substance by the operation of the devices in temperature cycled modes. The means for heating and controlling the temperature of the field effect gas sensor may comprise a substrate with resistive heater structure, e.g. a Pt microheater, onto which substrate the field effect gas sensor is mounted. Further, the means for heating and controlling the temperature of the field effect gas sensor may comprise a resistive heating structure deposited to the backside of the field effect gas sensor. The field effect gas sensor may also include a temperature sensor for measurement of the device temperature during operation, e.g. by an integrated diode structure or a Pt100 temperature sensor mounted to the heater and/or the field effect gas sensor.

The field effect gas sensor may be in a flip chip configuration. A flip chip configuration refers to a configuration of the field effect gas sensor in which the sensor may be attached directly to e.g. a circuit board or headers without any wire bonding. Thus, a flip chip configuration facilitates easier contacting and mounting to e.g. headers and/or circuit boards.

A flip chip configuration may be achieved by a contact layer on a part of the at least one electrical contact. The contact layer may be of a Pt film or a double layer of Ti/Au or Ti/Pt to facilitate wire or flip chip bonding. The flip chip configuration may also be achieved by using e.g. gold or platinum bumps for the interconnections so that when the field effect gas sensor is turned upside down, the at least one ohmic contact and/or the at least one electrical contact may be connected to some kind of substrate, circuit board or header via these gold or platinum bumps. The board to which the field effect gas sensor may be connected can be of e.g. a printed alumina (Al₂O₃) substrate or an LTCC structure or any other high temperature resistant material suitable for printing/deposition of interconnections. If the field effect gas sensor is configured in a flip chip configuration, it is much more resistant to vibrations and exposure to high temperatures compared to if the sensor is connected with wire bonds to e.g. circuit boards. Therefore, a flip chip configuration may be more suitable in certain applications.

The field effect gas sensor may be operable at temperatures ranging from about 100-700 °C, such as 100-600 °C, such as 100-450 °C, such as 150-300 °C, such as 250-450 °C..

The field effect gas sensor may be operable in a corrosive environment. A corrosive environment refers to an environment that may cause material to corrode by chemical reactions, such as oxidation.

A field effect gas sensor operable at temperatures within the ranges specified above and/or operable in a corrosive environment may be suitable for applications where the field effect gas sensor is subjected to harsh environments. Such applications may include on-line monitoring of flue gases from power plants, combined heat and power plants, local and domestic heating systems as well as exhaust from vehicles for control of the combustion of solid, liquid and/ or gaseous fuels, monitoring and control of after-treatment systems in both power plants and heating systems as well as vehicles, and on-board diagnostics of e.g. after-treatment systems.

At least one filter may be applied to the field effect gas sensor, e.g. in the form of molecular sieves or catalytic filters, either directly to the field effect gas sensor or as part of the mounting/ packaging of the field effect gas sensor, for enhanced selectivity or reduced sensitivity to certain substances. The field effect gas sensors according to the present disclosure may also be mounted/packaged in a packaging/housing suitable to the application in order to protect the field effect gas sensor as well as electronic connections, circuit boards etc. from physical impact from e.g. large soot or ash particles, large water drops etc. The packaging can be of any material and in any shape/design suitable to the application, examples include, but are not limited to, stainless steel, aluminium, alumina (Al₂O₃), other ceramics, AlSiC, copper (Cu), copper/tungsten alloys (Cu/W) and nickel/iron/cobalt alloys (Ni/Fe/Co).

The field effect gas sensor may be used for EGR (Exhaust Gas Recirculation) applications.

EGR (Exhaust Gas Recirculation) refers to the process whereby part of the exhaust gases is returned (and mixed with air) to the cylinders of a combustion engine. The recirculation of exhaust reduces the peak temperature of the combustion, and thus the nitrogen oxide emissions from an engine. However, in EGR, the oxygen concentration of the air/exhaust gas mixture must be measured and the recirculation must be adjusted for optimal combustion. The oxygen concentration must thus be measured in an environment that is highly corrosive and can contain droplets of condensed water as well as soot particles. Therefore, the use of the field effect gas sensor according to any aspects or embodiments of the invention in EGR would facilitate implementation of EGR in combustion engines and thus result in lower nitrogen oxide emissions.

A material substantially chemically inert to hydrogen may be used in a field effect gas sensor. A material substantially chemically inert to hydrogen is referred to as described above. The use of a material substantially chemically inert to hydrogen in a field effect gas sensor may decrease the sensitivity towards hydrogen or hydrogen-containing substances in the ambient, thus increasing the selectivity towards at least one non-hydrogen containing substance in a gas comprising hydrogen or hydrogen-containing substances.

The material substantially chemically inert to hydrogen may comprise MgO.

### Brief description of the drawings

Preferred embodiments of the invention will now be described in greater detail with reference to the accompanying drawings, in which
Fig. 1 illustrates a field effect gas sensor of the MOSFET/MISFET type according to an embodiment of the present invention;
Fig. 2 illustrates a field effect gas sensor of the MOS capacitor type according to an embodiment of the present invention;
Fig. 3 illustrates a field effect gas sensor of the Schottky diode type according to an embodiment of the present invention;
Fig. 4 illustrates an example of a suitable means for electrically connecting and heating the field effect gas sensor according to an embodiment of the present invention; Fig. 4A shows a front view, Fig. 4B shows a backside view and Fig. 4C shows a side view, in which a field effect gas sensor of the present invention is mounted to the suitable means for electrically connecting and heating;
Fig. 5 illustrates an example of an encapsulated field effect gas sensor according to an embodiment of the present invention; and
Fig. 6 illustrates an example of a configuration for detection of at least one non-hydrogen containing substance in a gas flow comprising hydrogen or hydrogen-containing substances using the field effect gas sensor according to an embodiment of the present invention.

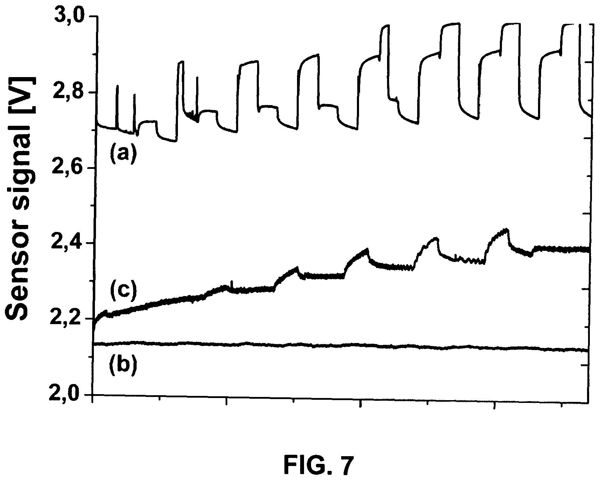

Fig. 7 displays the sensor signal from a O₂ sensor comprising a hydrogen inert insulating layer of MgO and the sensor signal from a sensor without such hydrogen inert insulating layer of MgO. Graph (a) shows the sensor signal from the O₂ sensor without MgO during exposure to pulses of CO and H₂ (ranging in concentration from 250 to 1250 ppm) in a background of 5% oxygen in nitrogen. Graph (b) shows the sensor signal from the O₂ sensor comprising MgO during exposure to the same pulses as in graph (a). Graph (c) shows the sensor signal from the O₂ sensor comprising MgO during exposure to pulses of 1, 2, 5, 10, 15% oxygen in a background of nitrogen.

### Description of preferred embodiments

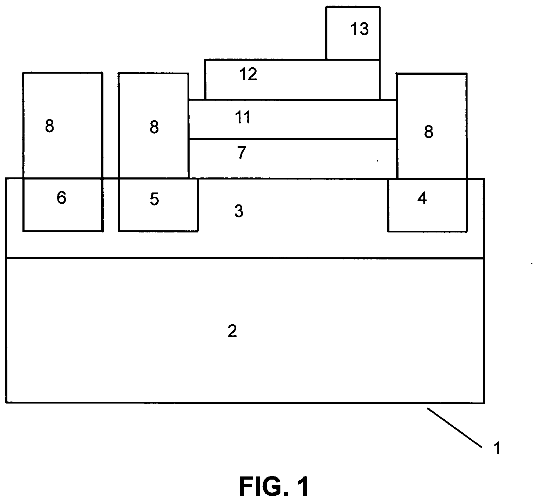

Fig. 1 displays an example of a field effect gas sensor of the MOSFET/ MISFET type (1) according to an embodiment of the present disclosure. The field effect gas sensor of the MOSFET/ MISFET type (1) comprises a semiconductor layer (2) of e.g. n-type doped SiC. On the semiconductor layer (2), an epilayer (3) (also of SiC), of p-type (doping concentration 5-10¹⁵/cm³) is grown to a thickness of approximately 10 µm. In the epilayer, (3) doped regions are created e.g. by ion implantation to form a drain region (4) of n-type, a source region (5) of n-type and a substrate region (6) of p-type (doping concentration approximately 1·10²⁰/cm³). On top of the epilayer (3) an electron insulating layer (7) is grown, consisting of e.g. a thermally grown SiO₂ layer to an approximate thickness of 500 Å, and an LPCVD deposited layer of silicon nitride (Si₃N₄) of approximate thickness 250 Å, which is densified to create a thin layer of silicon dioxide on top of the nitride, typically 50 Å. Three different ohmic contacts (8) to the source (5), drain (4) and substrate regions (6) of the epilayer (3) are then created. The ohmic contacts (8) may be processed by first etching the electron insulating layer (7) (e.g. using standard photo-lithographic patterning and wet etching techniques or dry etching techniques such as reactive ion etching) over the drain region (4) of n-type, the source region (5) of n-type and over the substrate region (6). Onto the implanted areas where the electron insulating layer has been removed the ohmic contacts (8) may then be created by the following process; deposition of Nickel (Ni) to an approximate thickness of 500 Å followed by rapid thermal annealing in argon at 950°C and then deposition of, approximately 500 Å tantalum silicide (TaSi₂) and 4000 Å platinum (Pt) or optionally 500 a titanium (Ti) and at least 2500 Å gold (Au). Onto the electron insulating layer (7), in between the ohmic contact to the source (5) and the ohmic contact to the drain (4), a layer comprising a material substantially chemically inert to hydrogen or hydrogen-containing substances (11), e.g. a MgO layer, is deposited e.g. by RF or reactive sputtering in an oxygen ambient, or evaporation, to a thickness of approximately 500Å. Onto at least a part of the layer comprising a material substantially chemically inert to hydrogen or hydrogen-containing substances (11), an electrical contact (12), which may be a gate contact when the field effect gas sensor is of MOSFET/ MISFET type, is created, comprising a thin film of a catalytic material, such as Ir, and/or a material capable of absorbing one or more of the gaseous substances of interest, such as RuOₓ or BaCoO₃. At least a part of the electrical contact (12) may be deposited by sputtering, in the case of Ru in an oxygen ambient, or evaporation to a thickness of approximately 100-500 Å. On top of the electrical gate contact (12) a thin, discontinuous layer of a catalytic material, e.g. 25Å Pt, may be deposited. Part of the electrical gate contact (12) may be covered with a contact layer (13) of a Pt film of a thickness of approximately 4000 Å or a double layer of Ti/Au (500 Å/2500 Å) or Ti/Pt (100 Å/4000 Å) to facilitate wire or flip-chip bonding. Adsorption of the at least one non-hydrogen containing substance of interest on the electrical gate contact (12) induce, either directly or through reactions with adsorbed oxygen anions, a change in the electric field in the semiconductor and thus a change in conductance in the channel between the source (5) and drain (4) regions. The voltage over the field effect gas sensor of the MOSFET/ MISFET type when keeping a constant current through the gas sensor thus reflects the presence and/ or ambient concentration of the at least one non-hydrogen containing substance.

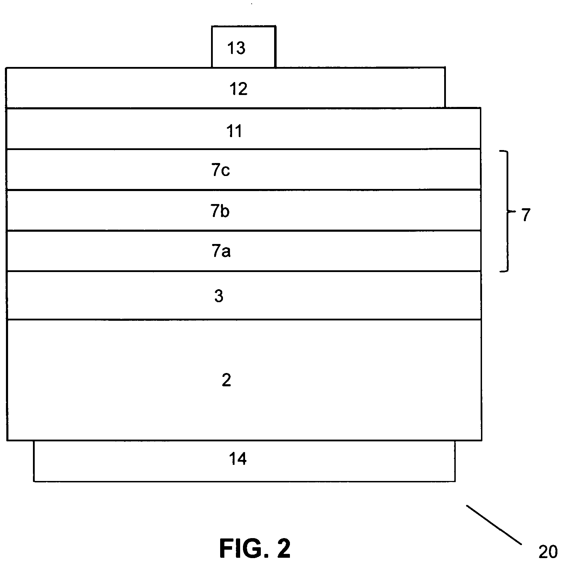

Fig. 2 displays an example of a field effect gas sensor of MOS capacitor type (20) according to an embodiment of the present disclosure. The field effect gas sensor of MOS capacitor type (20) has a semiconductor layer (2) of SiC, being of n-type semi-insulating material, onto which an epilayer (3) of n-type and of approximately 5 µm thickness, is grown. On top of the epilayer (3) an electron insulating layer (7) is created. The electron insulating layer (7) comprises a stack of three insulators (7a, 7b and 7c) consisting of a thermally grown oxide (SiO₂) (7a) and an LPCVD deposited and densified silicon nitride (Si₃N₄) (7b), the latter also resulting in a thin silicon dioxide film (7c) on top of the nitride, to an approximate total thickness of the electron insulating layer (7) of 800 Å. Onto the electron insulating layer (7) a layer comprising a material substantially chemically inert to hydrogen or hydrogen-containing substances (11), e.g. a MgO layer, is deposited. The MgO layer may be deposited by RF or reactive sputtering, or evaporation, to a thickness of approximately 500 Å. Further, a backside ohmic contact (14), e.g. comprising of a layer of sputter-deposited nickel (Ni), approximately 1000 Å in thickness and annealed at 950 °C in argon, and 500 Å of sputter-deposited tantalum silicide (TaSi₂) and approximately 4000 Å platinum (Pt), is created to the semiconductor layer (2). Onto a part of the layer comprising a material substantially chemically inert to hydrogen or hydrogen-containing substances (11), an electrical contact (12), consisting of a thin film of a catalytic material, such as Ir, and/or a material capable of absorbing one or more of the gaseous substances of interest, such as RuOₓ or BaCoO₃, may be deposited by reactive sputtering in an oxygen ambient, or by evaporation to a thickness of approximately 100-250 Å. As a top of the electrical contact (12), a thin, discontinuous layer of a catalytic metal, e.g. 25 Å Pt, may be deposited. On a part of the electrical contact (12) a contact layer (13) is created, consisting of a Pt film of a thickness of approximately 4000 Å or a double layer of Ti/Au (500 Å / 2500 Å) or Ti/Pt (100 Å / 4000Å) to facilitate wire or flip-chip bonding. Adsorption of the at least one non-hydrogen containing substance of interest on the electrical contact (12) induce, either directly or through chemical reactions e.g. with adsorbed oxygen anions, a change in material properties and/ or a change in the electric field in the semiconductor, thus changing the capacitance-voltage characteristics of the field effect gas sensor of MOS capacitor type. The bias voltage over the field effect gas sensor when keeping a constant capacitance over the sensor thus reflects the presence and/ or ambient concentration of the at least one non-hydrogen containing substance.

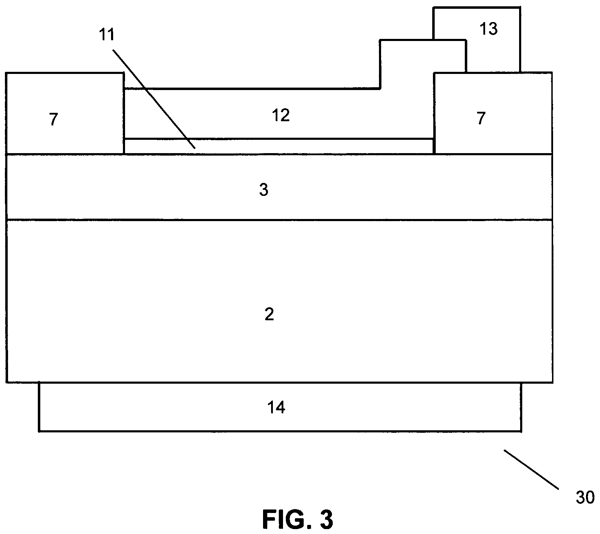

Fig. 3 displays an example of a field effect gas sensor of Schottky diode type (30) according to an embodiment of the present disclosure. The field effect gas sensor of Schottky diode type (30) has a semiconductor layer (2) of e.g. n-doped SiC. Onto the semiconductor layer (2) an epilayer (3) of n-type (e.g. doping concentration 3·10¹⁶/cm³) is grown to a thickness of approximately 10 µm. On top of the epilayer (3) an electron insulating layer (7) is created, consisting of a thermally grown oxide (SiO₂) layer to an approximate total thickness of approximately 800 Å. A backside ohmic contact (14), consisting of a layer of sputter-deposited nickel (Ni), approximately 1000 Å in thickness and annealed at 950 °C in argon, and 500 Å of sputter-deposited tantalum silicide (TaSi₂) and approximately 4000 Å platinum (Pt), is created to the semiconductor layer (2). The electron insulating layer (7) may be patterned by conventional photolithographic methods and wet etched in 50% HF so that a layer comprising a material substantially chemically inert to hydrogen or hydrogen-containing substances (11), e.g. a MgO layer, may be deposited in such way that it contacts both the epilayer (3) and the electron insulating layer (7). If the layer comprising a material substantially chemically inert to hydrogen or hydrogen-containing substances (11) is a MgO layer, it may be deposited to a thickness of approximately 50 Å by reactive sputtering of Mg in an oxygen ambient or by RF sputtering or evaporation of MgO. An electrical contact (12), consisting of a thin film of a catalytic material, such as iridium (Ir), or a material capable of absorbing one or more of the gaseous substances of interest, such as ruthenium oxide (RuOₓ) or barium cobalt oxide (BaCoO₃) may be deposited on top of the layer comprising a material substantially chemically inert to hydrogen or hydrogen-containing substances (11). At least a part of the electrical contact (12) may be deposited by sputtering, in the case of ruthenium (Ru) in an oxygen ambient, or by evaporation to a thickness of approximately 100-500 Å. A contact layer (13) is created on top of at least part of the electrical contact by sputter-deposition of a Pt film of a thickness of approximately 4000 Å or a layer of 100 Å titanium (Ti) followed by a layer of 4000 Å gold (Au), to facilitate wire or flip-chip bonding. The contact layer (13) may also cover a part of the electron insulating layer (7). Adsorption of the at least one non-hydrogen containing substance of interest on the electrical contact (12) induce, either directly or through reactions with adsorbed oxygen anions, a change in the Schottky barrier, thus changing the current of the field effect gas sensor of Schottky diode type. The bias voltage over the field effect gas sensor when keeping a constant current over the sensor thus reflects the presence and/ or ambient concentration of the at least one non-hydrogen containing substance of interest.

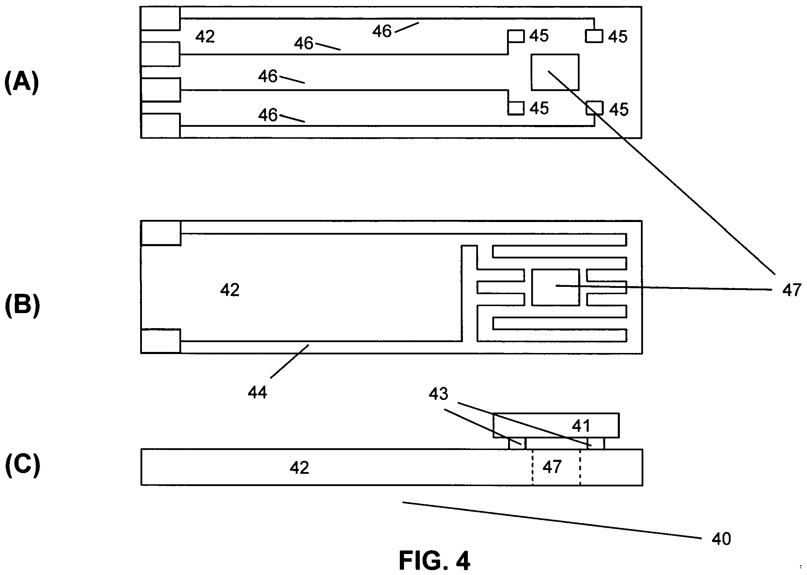

Fig. 4 displays an example of a suitable means (40) for electrically connecting and heating the field effect gas sensor of the present disclosure. An alumina substrate (42) (or a substrate of some other suitable material) has connector lines (46) and contact pads (45) printed on the front side and a resistive-type heater line (44) on the backside. The field effect gas sensor (41) is flipped upside-down and bumps (43) of e.g. gold or platinum connect the field effect gas sensor (41) to the contact pads (45) and connector lines (46) printed on the alumina substrate. An opening (47) is created in the alumina substrate just above the electrical contact (the gate contact in transistor devices) of the field effect gas sensor (41) to allow the ambient gas mixture to reach the electrical contact of the field effect gas sensor (41). The resistor structure (44) is printed on the backside of the alumina substrate (42) to facilitate heating of the sensor device. All connector lines (46) are printed in such a way that they can be easily contacted at the end of the alumina substrate by e.g. a clamp contact.

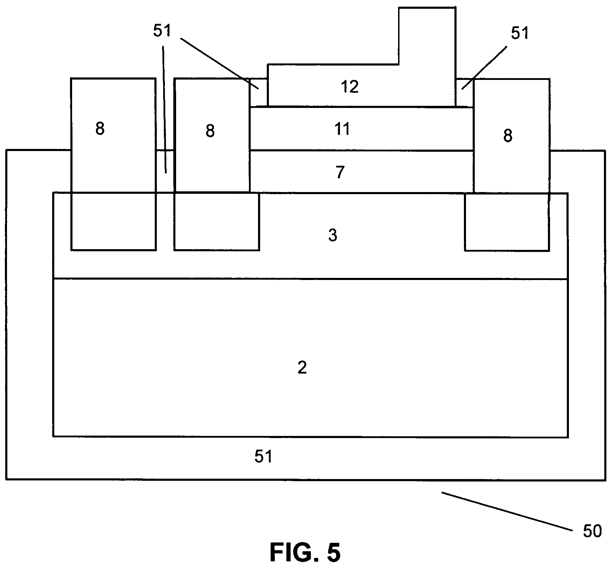

Fig. 5 displays an example of a field effect gas sensor of the present disclosure comprising means for encapsulation (50). The semiconductor layer (2), the epilayer (3), the electron insulating layer (7) and the layer comprising a material substantially chemically inert to hydrogen or hydrogen-containing substances (11) are covered with an encapsulation layer (51) of a suitable material, e.g. Si₃N₄ or SiO₂. The ohmic contacts (8) and the electrical contact (12) are however in contact with the ambient to facilitate detection of at least one non-hydrogen containing substance of interest in a gas comprising hydrogen or hydrogen-containing substances.

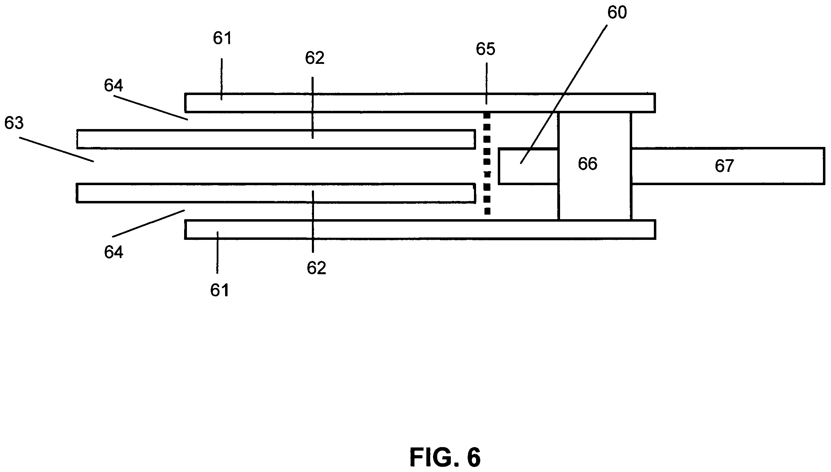

Fig. 6 displays an example of a configuration for detection of at least one non-hydrogen containing substance in a gas flow comprising hydrogen or hydrogen-containing substances using a field effect gas sensor (60) according to the present disclosure. The configuration comprising the field effect gas sensor (60) is mounted in the gas flow of interest, e.g. in a tail pipe, a flue gas channel, a chimney etc. The field effect gas sensor (60) is placed inside an outer tube (61) a short distance from the end of an inner tube (62). The inner tube (62) is of smaller diameter than the outer tube (61) and disposed within the outer tube (61) such that there is a gap between the inner (62) and the outer (61) tube. Furthermore, the inner tube (62) extends outside the outer tube (61) at the end opposite to the location of the field effect gas sensor (60). In between the end of the inner tube (62) and the field effect gas sensor (60) a coarse filter (65) is applied such that it spans the cross section of the outer tube (61). The outer (61) and inner (62) tubes are assembled such that the gas mixture of interest can pass in through the outer tube opening (64), come into contact with the field effect gas sensor (60) and exit through the opening of the inner tube (63). The outer tube (61) is also supplied with a gas-tight thermal barrier (66) and means for electrically connecting the sensor device (67) as well as a thread for screwing it into place.

### Experimental example

An experiment of comparing a field effect sensor sensitive to O₂ comprising a hydrogen inert insulating layer of MgO with a field effect sensor sensitive to O₂ without such hydrogen inert layer was performed. Both field effect sensors were subjected to pulses of CO and H₂ (ranging in concentration from 250 to 1250 ppm) in a background of 5% oxygen in nitrogen. Further, the sensor comprising the MgO layer was also subjected to pulses of 1, 2, 5, 10, 15% oxygen in a background of nitrogen. The results are displayed in Fig. 7.

It was evident from these results that an O₂ sensor comprising a hydrogen inert insulating layer of MgO was not sensitive to the H₂ pulses (graph (b) in Fig. 7), whereas the O₂ sensor without the hydrogen inert insulating layer of MgO was severely affected by the H₂ pulses (graph (a) in Fig. 7). Further, it was seen that the O₂-sensor comprising a hydrogen inert insulating layer of MgO responded nicely when subjected to the pulses of O₂ (graph (c) in Fig. 7).

Consequently, the results clearly shows that a field effect O₂ sensor comprising a hydrogen inert insulating layer of MgO has a high specificity towards 02 and is very insensitive to H₂ contaminations in the ambient compared to a sensor without such MgO layer.

## Claims

1. A field effect gas sensor (1) for detection of at least one non-hydrogen containing substance in a gas comprising hydrogen or hydrogen-containing substances, said field effect gas sensor comprising:
- a semiconductor layer (2, 3) having a surface;
- at least one ohmic contact (8) contacting said semiconductor (2, 3);
- a first electron insulating layer (7) covering at least a part of said surface of said semiconductor layer (2, 3);
- a second insulating layer (11) contacting at least one of said first electron insulating layer (7) and said semiconductor layer (2, 3);
- at least one electrical contact (12) contacting said second insulating layer (11) and comprising a conducting, semi-conducting and/or ion conducting layer;
wherein said field effect gas sensor is configured so interaction of said at least one non-hydrogen containing substance with at least part of said at least one electrical contact affects the work function of said conducting, semi-conducting and/or ion conducting layer and/or the electric field in said semiconductor layer in such way that the current-voltage (I-V) or the capacitance-voltage (C-V) characteristics of said field effect gas sensor are changed, wherein said change in I-V or C-V characteristics provide information about the presence of said at least one non-hydrogen containing substance in said gas,
**characterized in that** the second insulating layer comprises MgO.

2. The field effect gas sensor according to claim 1, wherein said I-V or said C-V characteristics are changed such that a voltage applied over said at least one ohmic contact to keep a constant current through or a constant capacitance over said semiconductor layer increases or decreases, wherein said increase or decrease provides information about the presence of said at least one non-hydrogen containing substance in said gas.

3. The field effect gas sensor according to claim 1 or 2, wherein the field effect gas sensor (1) is selected from the group consisting of MIS/MOS (Metal Insulator Semiconductor/ Metal Oxide Semiconductor) capacitors, Schottky diodes and field effect transistors.

4. The field effect gas sensor according to claim 3, wherein the field effect transistor is selected from the group consisting of Metal Oxide Semiconductor Field Effect Transistors (MOSFET), Metal Insulator Semiconductor Field Effect Transistors (MISFET), Metal Semiconductor Field Effect Transistors (MESFET), Heterostructure Field Effect Transistors (HFET), and Metal Insulator Semiconductor Heterostructure Field Effect Transistors (MISHFET).

5. The field effect gas sensor according to any previous claim, wherein the at least one non-hydrogen containing substance is selected from CO, CO₂, NO, NO₂, N₂O, SO₂, SO₃ and O₂, or any combination thereof.

6. The field effect gas sensor according to any previous claim, wherein the semiconductor layer (2, 3) comprises a wide band gap semiconductor.

7. The field effect gas sensor according to claim 6, wherein the wide band gap semiconductor is selected from the group consisting of SiC, group III nitrides and alloys of group III nitrides, such as, GaN, Al_{X}Ga_{1-X}N, AlN, InN, In_{X}Ga₁-_{X}N and ZnO, or any combination thereof.

8. The field effect gas sensor according to any previous claim, wherein the first electron insulating layer (7) comprises a material selected from the group consisting of SiO₂, Al₂O₃, Ta₂O₅, and V₂O₅, or any combination thereof.

9. The field effect gas sensor according to any previous claim, wherein said conducting, semi-conducting and/or ion conducting layer of said electrical contact conducts ions of said at least one non-hydrogen containing substance.

10. The field effect gas sensor according to any previous claim, wherein the conducting, semi-conducting and/or ion conducting layer comprises a material selected from the group consisting of Pt, Ir, Ru, Rh, RuO₂, IrO₂, La_{1-X}Ba_{X}CoO₃, CeO₂, SnO₂, WO₃, TiO₂ and conducting ceramics, or any combination thereof.

11. The field effect gas sensor according to any previous claim, wherein said at least one electrical contact (12) comprises at least one catalytic material.

12. The field effect gas sensor according to any previous claim, wherein the field effect gas sensor further comprises at least one catalytic material contacting at least one of the first electron insulating layer, the second insulating layer or the at least one electrical contact (12).

13. The field effect gas sensor according to claim 11 or 12, wherein the at least one catalytic material is porous.

## Patentansprüche

1. Feldeffekt-Gassensor (1) zur Erkennung mindestens eines nicht wasserstoffhaltigen Stoffes in einem Gas, das Wasserstoff oder wasserstoffhaltige Stoffe umfasst, wobei der Feldeffekt-Gassensor Folgendes umfasst:
- eine Halbleiterschicht (2, 3) mit einer Oberfläche;
- mindestens einen ohmschen Kontakt (8), der den Halbleiter (2, 3) kontaktiert;
- eine erste Elektronenisolierschicht (7), die mindestens einen Teil der Oberfläche der Halbleiterschicht (2, 3) abdeckt;
- eine zweite Isolierschicht (11), die mindestens eine der ersten Elektronenisolierschicht (7) und der Halbleiterschicht (2, 3) kontaktiert;
- mindestens einen elektrischen Kontakt (12), der die zweite Isolierschicht (11) kontaktiert und eine Leiter-, Halbleiter- und/oder lonenleiterschicht umfasst;
wobei der Feldeffekt-Gassensor derart konfiguriert ist, dass eine Wechselwirkung des mindestens einen nicht wasserstoffhaltigen Stoffes mit mindestens einem Teil des mindestens einen elektrischen Kontakts die Austrittsarbeit der Leiter-, Halbleiter- und/oder lonenleiterschicht und/der das elektrische Feld in der Halbleiterschicht derart beeinflusst, dass die Strom-Spannungs- (I-V) oder die Kapazitäts-Spannungs- (C-V) -Eigenschaften des Feldeffekt-Gassensors geändert werden, wobei die Änderung der I-V- oder C-V-Eigenschaften Informationen über die Gegenwart des mindestens einen nicht wasserstoffhaltigen Stoffes in dem Gas bereitstellt, **dadurch gekennzeichnet, dass** die zweite Isolierschicht MgO umfasst.

2. Feldeffekt-Gassensor nach Anspruch 1, wobei die I-V- oder die C-V-Eigenschaften derart verändert werden, dass eine Spannung, die über dem mindestens einen ohmschen Kontakt angelegt wird, um einen konstanten Strom durch oder eine konstante Kapazität über der Halbleiterschicht zu halten, zu- oder abnimmt, wobei die Zu- oder Abnahme Informationen über die Gegenwart des mindestens einen nicht wasserstoffhaltigen Stoff in dem Gas bereitstellt.

3. Feldeffekt-Gassensor nach Anspruch 1 oder 2, wobei der Feldeffekt-Gassensor (1) ausgewählt ist aus der Gruppe bestehend aus MIS/MOS-(Metallisolatorhalbleiter/Metalloxidhalbleiter)-Kondensatoren, Schottky-Dioden und Feldeffekttransistoren.

4. Feldeffekt-Gassensor nach Anspruch 3, wobei der Feldeffekttransistor ausgewählt ist aus der Gruppe bestehend aus Metalloxidhalbleiter-Feldeffekttransistoren (MOSFET), Metallisolatorhalbleiter-Feldeffekttransistoren (MISFET), Metallhalbleiter-Feldeffekttransistoren (MESFET), Heterostruktur-Feldeffekttransistoren (HFET) und Metallisolatorhalbleiter-Heterostruktur-Feldeffekttransistoren (MISHFET).

5. Feldeffekt-Gassensor nach einem der vorhergehenden Ansprüche, wobei der mindestens eine nicht wasserstoffhaltige Stoff ausgewählt ist aus CO, CO₂, NO, NO₂, N₂O, SO₂, SO₃ und O₂ oder einer beliebigen Kombination davon.

6. Feldeffekt-Gassensor nach einem der vorhergehenden Ansprüche, wobei die Halbleiterschicht (2, 3) einen Halbleiter mit großer Bandlücke umfasst.

7. Feldeffekt-Gassensor nach Anspruch 6, wobei der Halbleiter mit großer Bandlücke ausgewählt ist aus der Gruppe bestehend aus SiC, Nitriden der Gruppe III und Legierungen von Nitriden der Gruppe III wie GaN, AlₓGa₁₋ₓN, AlN, InN, InₓGa₁₋ₓN und ZnO oder einer beliebigen Kombination davon.

8. Feldeffekt-Gassensor nach einem der vorhergehenden Ansprüche, wobei die erste Elektronenisolierschicht (7) ein Material umfasst, das ausgewählt ist aus der Gruppe bestehend aus SiO₂, Al₂O₃, Ta₂O₅ und V₂O₅ oder einer beliebigen Kombination davon.

9. Feldeffekt-Gassensor nach einem der vorhergehenden Ansprüche, wobei die Leiter-, Halbleiter- und/oder lonenleiterschicht des elektrischen Kontakts Ionen des mindestens einen nicht wasserstoffhaltigen Stoffs leitet.

10. Feldeffekt-Gassensor nach einem der vorhergehenden Ansprüche, wobei die Leiter-, Halbleiter- und/oder lonenleiterschicht ein Material umfasst, das ausgewählt ist aus der Gruppe bestehend aus Pt, Ir, Ru, Rh, RuO₂, IrO₂, La₁₋ₓBaₓCoO₃, CeO₂, SnO₂, WO₃, TiO₂ und leitende Keramiken oder einer beliebigen Kombination davon.

11. Feldeffekt-Gassensor nach einem der vorhergehenden Ansprüche, wobei der mindestens eine elektrische Kontakt (12) mindestens ein katalytisches Material umfasst.

12. Feldeffekt-Gassensor nach einem der vorhergehenden Ansprüche, wobei der Feldeffekt-Gassensor ferner mindestens ein katalytisches Material umfasst, das mindestens eine der ersten Elektronenisolierschicht, der zweiten Isolierschicht oder des mindestens einen elektrischen Kontakts (12) kontaktiert.

13. Feldeffekt-Gassensor nach Anspruch 11 oder 12, wobei das mindestens eine katalytische Material porös ist.

## Revendications

1. Détecteur de gaz à effet de champ (1) pour la détection d'au moins une substance ne contenant pas d'hydrogène dans un gaz comprenant de l'hydrogène ou des substances contenant de l'hydrogène, ledit détecteur de gaz à effet de champ comprenant :
- une couche de semi-conducteur (2, 3) dotée d'une surface ;
- au moins un contact ohmique (8) en contact avec ledit semi-conducteur (2, 3) ;
- une première couche isolante à électron (7) couvrant au moins une partie de ladite surface de couche de semi-conducteur (2, 3) ;
- une seconde couche isolante (11) en contact avec au moins une de ladite première couche isolante à électron (7) et de ladite couche de semi-conducteur (2, 3) ;
- au moins un contact électrique (12) en contact avec ladite seconde couche isolante (11) et comprenant une couche conductrice, semi-conductrice et/ou conductrice ionique ;
ledit détecteur de gaz à effet de champ étant conçu pour qu'une interaction de ladite au moins une substance ne contenant pas d'hydrogène avec au moins une partie de l'au moins un contact électrique affecte la fonction opératoire de ladite couche conductrice, semi-conductrice et/ou juste conductrice ionique et du champ électrique dans ladite couche semi-conductrice de telle sorte que les caractéristiques de voltage électrique (I-V) ou de voltage de capacité électrique (C-V) dudit détecteur de gaz à effet de champ changent, ledit changement de caractéristiques d'I-V ou de C-V donnant des informations sur la présence de ladite au moins une substance ne contenant pas d'hydrogène dans ledit gaz,
**caractérisé en ce que** la seconde couche isolante contient du MgO.

2. Détecteur de gaz à effet de champ selon la revendication 1, dans lequel les caractéristiques desdites I-V ou C-V sont modifiées de manière à ce qu'une tension appliquée par ledit au moins un contact ohmique afin de garder un courant constant augmente ou diminue, ladite augmentation ou diminution donnant des informations sur la présence de ladite au moins une substance ne contenant pas d'hydrogène dans ledit gaz.

3. Détecteur de gaz à effet de champ selon la revendication 1 ou 2, dans lequel le détecteur de gaz à effet de champ (1) est sélectionné dans le groupe composé de capacités électriques MIS/MOS (semi-conducteur isolant de métal/semi-conducteur d'oxyde de métal), diodes Schottky et transistors à effet de champ.

4. Détecteur de gaz à effet de champ selon la revendication 3, dans lequel le transistor à effet de champ est sélectionné dans le groupe composé de transistors à effet de champ à semi-conducteur d'oxyde de métal (MOSFET), transistors à effet de champ isolant de métal (MISFET), transistor à effet de champ à semi-conducteur de métal (MESFET), transistors à effet de champ à hétérostructure (HFET) et transistors à effet de champ à hétérostructure à semi-conducteur isolant de métal (MISHFET).

5. Détecteur de gaz à effet de champ selon l'une quelconque des revendications précédentes, dans lequel l'au moins une substance ne contenant pas d'hydrogène est sélectionnée parmi les CO, CO₂, NO, NO₂, N₂O, SO₂, SO₃ et O₂ ou toutes leurs combinaisons.

6. Détecteur de gaz à effet de champ selon l'une quelconque des revendications précédentes, dans lequel la couche semi-conductrice (2, 3) comprend un semi-conducteur à large bande énergétique.

7. Détecteur de gaz à effet de champ selon la revendication 6, dans lequel le semi-conducteur à large bande énergétique est sélectionné dans le groupe composé de SiC, nitrure de groupe III et alliages de groupe III tels que GaN, AlₓGa₁₋ₓN, AlN, InN, InₓGa₁₋ₓN et ZnO ou toutes leurs combinaisons.

8. Détecteur de gaz à effet de champ selon l'une quelconque des revendications précédentes, dans lequel la première couche isolante à électrons (7) comprend un matériau sélectionné dans le groupe composé de Si02, Al₂O₃, Ta₂O₅ et V₂O₅ ou toutes leurs combinaisons.

9. Détecteur de gaz à effet de champ selon l'une quelconque des revendications précédentes, dans lequel ladite couche conductrice, semi-conductrice et/ou conductrice ionique dudit contact électrique conduit des ions de ladite au moins une substance ne contenant pas d'hydrogène.

10. Détecteur de gaz à effet de champ selon l'une quelconque des revendications précédentes, dans lequel la couche conductrice, semi-conductrice et/ou conductrice ionique comprend un matériau sélectionné dans le groupe composé de Pt, Ir, Ru, Rh, RuO₂, IrO₂, Lai-ₓBaₓCoO₃, Ce02, Sn02, WO₃, Ti02 et des céramiques conductrices ou toutes leurs combinaisons.

11. Détecteur de gaz à effet de champ selon l'une quelconque des revendications précédentes, dans lequel ledit au moins un contact électrique (12) comprend au moins un matériau catalytique.

12. Détecteur de gaz à effet de champ selon l'une quelconque des revendications précédentes, dans lequel le détecteur de gaz à effet de champ comprend en outre au moins un matériau catalytique en contact avec au moins un élément parmi la première couche isolante à électrons, la seconde couche isolante ou l'au moins un contact électrique (12).

13. Détecteur de gaz à effet de champ selon la revendication 11 ou 12, dans lequel l'au moins un matériau catalytique est poreux.
